# EUROPÄISCHE PATENTSCHRIFT

(11) **EP 1 466 599 B1**
(45) Veröffentlichungstag und Bekanntmachung des Hinweises auf die Patenterteilung: **26.09.2007**
(21) Anmeldenummer: 04007307.4
(22) Anmeldetag: 26.03.2004
(51) Int. Cl.: A61K 31/282, A61K 9/08, A61K 47/26, A61P 35/00

(54) **Oxaliplatinlösungskonzentrate**
Concentrated solution of oxaliplatinum
Solution concentrée d'oxaliplatine

(30) Priorität: 28.03.2003 DE 10314377
(43) Veröffentlichungstag der Anmeldung: 13.10.2004
(73) Patentinhaber: STADA ARZNEIMITTEL AG, 61118 Bad Vilbel (DE)
(72) Erfinder: Schridde, Edgar, 30451 Hannover (DE); Merbach, Bernd, Dr., 30938 Burgwedel (DE); Gimmel, Stefan-Peter, Dr., 30655 Hannover (DE)
(74) Vertreter: Hamm, Volker

(56) Entgegenhaltungen:
- EP-A- 0 943 331
- WO-A-01/15691
- US-A- 6 153 646

## Beschreibung

Die vorliegende Erfindung betrifft pharmazeutische Zubereitungen von Oxaliplatin (I) zur Verabreichung auf parenteralem Weg. Oxaliplatin (*cis*-Oxalato-(*trans*-1,2-cyclohexandiamin)-platin(II); *cis*-Oxalato-(1,2-cyclohexandiamin)-platin(II), *trans*-1,2-Diaminocyclohexane oxaliplatinum; CAS Nr. 61825-94-3, Mr 397,3; C₈H₁₄N₂O₄Pt) ist eine erstmals im Jahr 2001 im Arzneibuch monographierte Substanz (Pharmeuropa Vol. 13, No 3, 2001, S. 585-588). Sie repräsentiert einen Platin (II)-Komplex mit jeweils einem Äquivalent *trans*-1,2-Diaminocyclohexan und Oxalsäure und hat die chemische Struktur 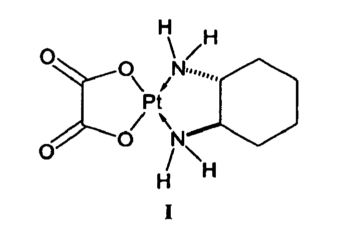 Oxaliplatin ist ein weißes, kristallines Pulver. Es ist löslich in Wasser, dagegen wenig löslich in Methanol und praktisch unlöslich in Ethanol. Es ist anti-neoplastisch wirksam und wird beispielsweise allein, aber auch in Kombination mit 5-Fluorouracil und/oder Folinsäure in der Behandlung des metastasierenden Colorectalcarcinoms therapeutisch eingesetzt. Die empfohlene Dosis in der Oxaliplatin-Therapie beträgt 85 mg/qm Körperoberfläche. Wie andere Platin-Verbindungen kann auch Oxaliplatin zytostatisch bei der therapeutischen Behandlung verschiedenster Krebsarten wie z.B. des Darmkrebses, des Eierstockkrebses oder des Krebses der oberen Atemwege eingesetzt werden. Dabei erfolgt die Verabreichung von Oxaliplatin in der Therapie der verschiedensten Krebserkrankungen intravenös, wodurch es erforderlich wird, den Wirkstoff in Form einer Lösung zur Verfügung zu stellen.

Die aus dem Stand der Technik bekannten, zur parenteralen Verabreichung benötigten Oxaliplatinlösungen werden unmittelbar vor der Verabreichung an den Patienten aus einem Lyophilisat des Oxaliplatins oder aus entsprechenden kristallinem bzw. amorphen Feststoffzubereitungen, die nicht durch Gefriertrocknungsverfahren hergestellt wurden, rekonstituiert. Die Verwendung von solchen Zubereitungen birgt aber wesentliche Nachteile. Zum einen ist das Herstellungsverfahren der Lyophilisate kompliziert und teuer, zum anderen stellt die Rekonstitution zusätzliche Arbeitsschritte dar und bedeutet für das Personal unerwünschte Risiken. Insbesondere kann es bei der Rekonstituierung der Arzneimittellösungen aus einer Trockensubstanz zum sogenannten "Spray-Back-Effect" kommen, durch den es zu einer weiteren Kontamination und Gefährdung des Personals kommen kann. Demgemäss muss sowohl bei der Herstellung des Lyophilisates, als auch bei seiner Rekonstitution jede Kontamination von Personal oder Inventar mit dem hochwirksamen Cytostatikum vermieden werden. Zudem werden an die zur Rekonstitution verwendeten Lösungsmittel hohe Ansprüche gestellt, und es darf sich bei Ihnen nicht um die ansonsten bei Injektionslösungen sehr üblichen Kochsalzlösungen handeln, da es dadurch zu einer Zersetzung des Oxaliplatinkomplexes kommt. Schließlich kann es auch durch andere Fehler in der Handhabung dieser Lyophilisate zu schwerwiegenden Problemen bei der Behandlung mit Oxaliplatin kommen, wie z.B. zu einer Abweichung der Wirkstoffkonzentration oder einer mikrobiellen Kontamination der Lösung. Aufgrund der vielfältigen Gefahren- und Fehlerquellen bei der Benutzung des lyophilisierten Wirkstoffs zur Herstellung von Oxaliplatinlösungen war es daher wünschenswert, gebrauchsfertige, im Folgenden auch ready-to-use-Lösungen genannte, Arzneimittellösungen zur Verfügung zu stellen.

Leider zeigt Oxaliplatin aber in Wasser, wenigstens bei geringen Konzentrationen unterhalb von 1 mg/ml, nur eine sehr geringe Stabilität. In diesem Zusammenhang und der gesamten weiteren Beschreibung der vorliegenden Erfindung ist "Stabilität der Lösung" immer als Stabilität des Oxaliplatin-Komplexes in Lösung zu verstehen, d.h. als langfristige relative Konstanz der Konzentration des Ausgangskomplexes nach Inlösungbringen. Die unzureichende Stabilität von Oxaliplatinlösungen in Wasser resultiert aus der Instabilität des Platinkomplexes selbst, dessen recht labile Liganden durch andere, stärkere bzw. reaktivere Nukleophile ausgetauscht werden können, so dass eine Zerstörung des Ausgangskomplexes resultiert. Deshalb ist die Wahl der Zusätze in Lösungen von Platinkomplexen grundsätzlich kritisch.

Es ist zum Beispiel bekannt, dass Chlorid-Anionen zur Zersetzung von Oxaliplatin führen, was auch der Grund dafür ist, dass Oxaliplatin-Lyophilisate nicht mit Kochsalzlösungen rekonstituiert werden können. Selbst das Hydroxid-Anion aus dem Lösungsmittel Wasser ist in der Lage, Liganden aus einem Platinkomplex zu substituieren, so dass labile Platinkomplexe und dementsprechend auch Oxaliplatin in wässriger Lösung stabilisiert werden müssen. Als durch die Anwesenheit von Hydroxid-Anionen verursachte Zerfalls- bzw. Reaktionsprodukte des Oxaliplatins (Oxalato-DACH-Platin) sind z.B. das Diaquo-DACH-Platin **(II)** und das Diaquo-DACH-Platin-Dimer **(III)** bekannt.

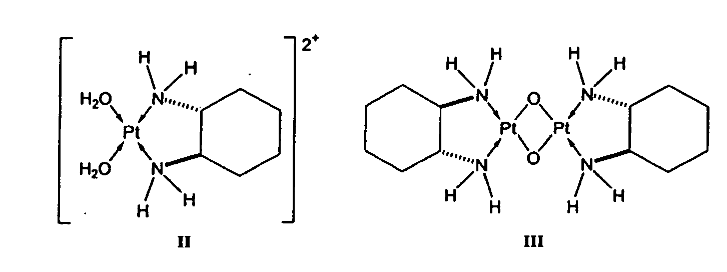

Zur Stabilisierung der Oxaliplatinkomplexe in wässriger Lösung kann die Konzentration bzw. die Aktivität der zersetzenden Anionen, z.B. des Hydroxid-Anions in wässrigen Lösungsmitteln, reduziert werden. Dazu wurde im europäischen Patent EP 0 774 963 vorgeschlagen, die Konzentration des Oxaliplatins auf mindestens 1 mg/ml zu erhöhen, wodurch sich gemäß dieses Dokuments ein saurer pH-Wert zwischen 4,5 und 6 einstellte, die Hydroxid-Anionen Konzentration also erniedrigt ist. Bei Nacharbeitung der Beispiele aus EP 0 774 963 wurden jedoch ausschließlich Lösungen mit pH-Werten größer 6,0 erhalten.

Ansonsten sind die in EP 0 774 963 beschriebenen wässrigen Oxaliplatin-Lösungen aber frei von jedwedem sauren oder alkalischen Mittel, Puffer oder anderen Zusatzstoffen, da deren Interaktion mit dem Oxaliplatin-Komplex und ihre Effekte auf dessen Stabilität nicht vorhersehbar waren. Nachteilig wirkt sich bei diesem Ansatz aus, dass Oxaliplatin in Wasser nur eine geringe Löslichkeit zeigt. Das daraus resultierende Potential einer unerwünschten Auskristallisation aus übersättigten Lösungen birgt neben einer Wirkstoffkonzentrationsabnahme die immanente Gefahr von ungelösten Partikeln, die bei ihrer Injektion und Infusion zu z.T. lebensbedrohlichen Embolien (Blutgefässverschlüssen) führen können.

Ein Ausweg aus dieser Problematik wurde im europäischen Patent EP 1 207 875 gezeigt, indem die Löslichkeit des Oxaliplatinkomplexes in Wasser durch Zusatz von 1,2-Propandiol, Glycerin, Maltit, Saccharose oder Inosit verbessert wurde. Leider besitzen diese Zusätze bei ihrer Verwendung zur Herstellung von injizierbaren Arzneimittelzubereitungen erhebliche Nachteile. So sind sie alle als Kohlenhydrate leicht verfügbare Energiequellen, die zu einer unerwünschten Entgleisung des Energiestoffwechsels führen können, insbesondere aufgrund des beim Oxaliplatintherapiekollektiv altersbedingt weitverbreiteten Diabetes mellitus. Zudem ist z.B. Inosit unter anderem ein physiologischer, intrazellulärer Zucker und sein Phosphat stellt einen essentiellen Bestandteil einer Signaltransduktionskaskade dar. Inosit wird als zusätzlicher Reifungsförderer oral und intravenös im Rahmen der experimentellen Therapie organunreifer Frühgeborener gegeben. Es birgt ferner das unerwünschte Potential neurologischer Nebenwirkungen. Inosit ist für injektabile parenterale Darreichungsformen außerhalb der oben genannten Therapien weder zugelassen, noch bekannt.

Auch die anderen im europäischen Patent EP 1 207 875 vorgestellten Hydroxyverbindungen eigenen sich kaum zur Herstellung parenteraler Arzneiformen. Sie alle haben den Nachteil, dass sie selbst nach Verdünnung mit z.B. 250-500 ml 5%iger Glucoselösung unverträgliche Injektionslösungen erzeugen können und intravenös gegeben unphysiologische Bestandteile mit unbekanntem toxikologischem Profil darstellen. Allen Hydroxyderivaten, deren Verwendung in EP 1 207 875 zur Herstellung von Oxaliplatin-Lösungen vorgeschlagen wurde, gehören nicht zu den Standard-Hilfsstoffen mit bekannten Nebenwirkungen die zur Verwendung in Injektabilia Anwendung finden. Üblicherweise werden diese Verbindungen nur in pharmazeutischen Zubereitungen für die äußerliche bzw. orale Verabreichung als Hilfsstoff verwendet.

Falls nicht anders angegeben, sind %-Angaben in vorliegender Anmeldung immer als Gewichtsprozent (Gew.-%) zu verstehen.

Aufgabe der vorliegenden Erfindung ist es damit, stabile, gebrauchsfertige Oxaliplatinlösungskonzentrate zur Verfügung zu stellen, die nicht die zuvor diskutierten Nachteile der vorbekannten Lösungen mit Kohlenhydratzusatz haben und in denen selbst bei OxaliplatinKonzentrationen von oberhalb 6 mg/ml der Wirkstoff nicht zur spontanen Ausfällung oder Kristallisation neigt. Eine weitere wesentliche Aufgabe dieser Erfindung ist es, vorteilhafte Verfahren zur Herstellung solcher Lösungen anzugeben.

In den der vorliegenden Erfindung zugrundeliegenden Untersuchungen zeigte sich überraschend, dass die erfindungsgemäßen Oxaliplatinkonzentrate nicht nur stabil gegen Auskristallisation bzw. spontaner Ausfällung des Wirkstoffs sind, sondern dass auch die Oxaliplatinstabilität selbst in diesen Konzentraten drastisch verbessert ist. So zeigen Oxaliplatinlösungen mit Konzentration von 10 mg/ml in 20% - 82% (w/v) wässrigen Glucoselösungen (entsprechend 22% - 90%, w/v Glucose Monohydrat), in denen sich ohne weitere Zusätze ein pH-Wert von etwa 5,5 einstellt, nur geringfügigen Abbau. Insbesondere zeigte sich in diesen Untersuchungen, dass mit höheren Glucosekonzentrationen die Bildung an unerwünschtem Diamino-DACH-Platin(II)-Dimer (III) verringert oder unterdrückt werden kann.

Da der Wirkstoff Oxaliplatin in Wasser mit ca. 7,5 mg/ml relativ schlecht löslich ist, und da der Vorgang seiner Auflösung bis zur klaren, sichtbar partikelfreien Lösung unter herkömmlichen Bedingungen langwierig ist und nur unter Wärmezufuhr (40°C), die ihrerseits die Zersetzung des Wirkstoffs fördert, deutlich beschleunigt werden kann, ist eine weitere Aufgabe der vorliegenden Erfindung, eine Methode zur Verfügung zu stellen, die es ermöglicht, den Wirkstoff unter besonders schonenden Bedingungen, insbesondere ohne Erwärmung bzw. bei niedrigen Temperaturen, zügig aufzulösen. Bei den der vorliegenden Erfindung zugrundeliegenden Untersuchungen zeigte sich überraschend (siehe Tabellen 1 und 2), dass zur Herstellung dieser erfindungsgemäßen Konzentrate mit Oxaliplatin Konzentrationen von 5 bis 50 mg/ml besonders kohlenhydrathaltige Lösungen als Lösungsmittel geeignet sind. Die Konzentration des Kohlenhydrats in den erfindungsgemäßen Konzentraten kann der gewünschten Oxaliplatin-Konzentration und dem zu verwendenden Kolenhydrat angepasst werden. So können je nach Kohlenhydrat, dessen Löslichkeit und der angestrebten Oxaliplatin-Konzentration Kohlenhydrat-Konzentrationen von ca. 20 mg/ml bis etwa 1000 mg/ml verwendet werden. Bei der aseptischen Herstellung (Auflösung, Filtration, Abfüllung und Zwischenlagerung) sollte schonend, möglicherweise unter erniedrigten Temperaturen gearbeitet werden.

**Tabelle 1: Auflösungsgeschwindigkeiten von Oxaliplatin zum Erhalt einer Konzentration von 5 mg/ml in Wasser und 5%iger Glucoselösung.**

| Prüflösung (pH nicht eingestellt) | 40°C | 20-25°C | 10-15°C |
|---|---|---|---|
| Oxaliplatin (c = 5 mg/ml) in Wasser f. Injektionen, | < 15 min | < 60 min | <420 min |
| Oxaliplatin (c = 5 mg/ml) in 5% Glucose | < 15 min | < 60 min | < 360 min |

**Tabelle 2: Auflösungsgeschwindigkeiten von Oxaliplatin zum Erhalt einer Konzentration von 10 mg/ml in Wasser und Glucoselösungen verschiedener Konzentration.**

| Prüflösung (pH nicht eingestellt) | 40°C | 20-25°C | 10-15°C |
|---|---|---|---|
| Oxaliplatin (c = 10 mg/ml) in Wasser f. Injektionszwecke | < 60 min | nicht möglich | nicht möglich |
| Oxaliplatin (c = 10 mg/ml) in 10% Glucose | < 30 min | < 90 min | < 420 min |
| Oxaliplatin (c = 10 mg/ml) in 20% Glucose | < 15 min | < 75 min | < 270 min |
| Oxaliplatin (c = 10 mg/ml) in 30% Glucose | < 15 min | < 60 min | < 240 min |
| Oxaliplatin (c = 10 mg/ml) in 40% Glucose | < 15 min | < 45 min | < 180 min |
| Oxaliplatin (c = 10 mg/ml) in 50% Glucose | < 15 min | < 45 min | < 150 min |

Wie in den Tabellen 1 und 2 vorgeführt wird durch den Zusatz eines Kohlenhydrats wie etwa Glucose das Lösungsvermögen verbessert. Während OxaliplatinKonzentrationen von 10 mg/ml in Lösungen ohne Kohlenhydrat nur bei stark erhöhten Temperaturen zu erzielen sind, ist eine solche Menge Wirkstoff in Glukoselösungen selbst bei relativ niedrigen Temperaturen leicht aufzulösen. Zum anderen verbessern sich die Auflösungsgeschwindigkeiten durch den Zusatz von Kohlenhydraten drastisch, wodurch die Herstellung der Lösungen erheblich vereinfacht wird.

Eine Änderung des pH-Wertes der Lösungen, von ca. 5,0 bis 6,5 auf einen Wert von pH 3,0 bis 6,0 mit Oxal-, Phoshor- oder Schwefelsäure bewirkte demgegenüber keine Verkürzung der Auflösungsdauer. Die vorliegenden Ergebnisse veranschaulichen eindrucksvoll, dass der Zusatz von bestimmten Kohlenhydraten bei der Herstellung von nicht pH-eingestellten Lösungskonzentraten die Auflösungsgeschwindigkeit von Oxaliplatin erhöht, insbesondere auch bei deutlich erniedrigten Temperaturen, die wiederum zur Verminderung der Zersetzung des Wirkstoffs beitragen. Erfindungsgemäß wird ein Temperaturbereich zur wirkstoffschonenden Auflösung des Oxaliplatins von 10 - 25°C bevorzugt, besonders bevorzugt werden Temperaturen von 10 - 20°C, ganz besonders 10 - 15°C.

Die erfindungsgemäße Lösung der oben beschriebenen Aufgabe liegt darin, eine Stabilisierung von Oxaliplatinlösungen durch den Zusatz eines Kohlenhydrats ausgewählt aus der Gruppe umfassend Galaktose, Fruktose, Maltose, sowie Dextrane (10-70). Die bevorzugte Konzentration des Kohlenhydrats in solchen Lösungen beträgt 10 bis 100 Gew-%; besonders bevorzugt werden 10 bis 50 Gew-%.

Neben der Erhöhung der Löslichkeit des Oxaliplatins und damit einhergehender Stabilisierung der Lösung selbst ermöglicht der Zusatz der vorgenannten Kohlenhydrate zu den erfindungsgemäßen Lösungen auch höhere Konzentrationen an Oxaliplatin, ohne dass es zu einer unerwünschten Ausfällung bzw. Auskristallisation des Wirkstoffs kommt. Zudem assistieren die erfindungsgemäßen Kohlenhydratzusätze in der Erniedrigung der Hydroxidionenkonzentration, da sie als Verbindungen selbst in der Lage sind, den pH-Wert der Lösung zu erniedrigen, wie sich anhand der in Tabelle 3 zusammengefassten Untersuchungsergebnisse ergibt.

**Tabelle 3: Abhängigkeit des pH-Werts wässriger Glucoselösungen von der Glucosekonzentration**

| Glucose-Konzentration | experimentell ermittelter pH-Werte von Glucose gelöst in Wasser (20-25°C) |
|---|---|
| 5% Glucose | pH 6,2 |
| 10% Glucose | pH 6,1 |
| 20% Glucose | pH 5,8 |
| 30% Glucose | pH 5,7 |
| 40% Glucose | pH 5,5 |
| 50% Glucose | pH 5,3 |

Die erfindungsgemäßen Lösungen können ohne weiteren Zusatz einer Säure sein oder durch Zusatz einer Säure stabilisiert sein. Gerade bei Wirkstoffkonzentrationen von mehr als 5 mg/ml ist der Zusatz eines Kohlenhydrats als Löslichkeitsverbesserer angezeigt.

### Detaillierte Beschreibung der erfindungsgemäßen Lösungskonzentrate

Wie eingangs beschrieben, beträgt die empfohlene Dosis in der Oxaliplatin-Therapie 85 mg/qm Körperoberfläche. Bisher stehen hierfür nur aus einem Lyophilisat rekonstituierte Lösungen mit einem Gehalt von 5 mg/ml zur Verfügung. Bei einer durchschnittlichen Dosis von 150 mg Oxaliplatin/1,8 qm Körperoberfläche bedeutet dies, ein rekonstituiertes Lösungsvolumen von 30 ml mit 250 bis 500 ml 5 %iger Glucoselösung verdünnen zu müssen. Aus Betriebskostengründen wäre es von Vorteil, für die Lagerung von Arzneimitteln möglichst kleine Packungsgrößen (z.B. Durchstechflaschen) zu verwenden, ebenso ist es aus Gründen der Erzielung geringerer Betriebskosten und einer sichereren Handhabung von Vorteil, mit möglichst kleinen Lösungsvolumina zu hantieren. Diese zusätzlichen Vorteile bieten die erfindungsgemäßen gebrauchsfertigen Infusionslösungskonzentrate.

In den für die vorliegende Erfindung ausgeführten Experimenten zeigte sich hierzu, dass Oxaliplatin in wässriger Lösung mit Konzentrationen von maximal 7 bis 8 mg/ml (bei 25° C, Tabelle 4, erster Eintrag) löslich ist. Unter Verwendung von weiteren gelösten, tonisierenden, den pH-Wert einstellenden, puffernden oder konservierenden Hilfsstoffen wird nur noch eine maximale Konzentration von maximal 7 mg/ml Oxaliplatin in Wasser erreicht. Diese geringe Löslichkeit mit dem weiteren Gefahrenpotential einer unerwünschten Auskristallisation aus übersättigter Lösung bei geringeren Lagertemperaturen als 25° Celsius bedingt eine für die pharmazeutische Praxis maximale Oxaliplatin-Konzentration von 5 mg/ml. Um aber die oben genannten zusätzlichen Vorteile eines Infusionslösungskonzentrats mit z.B. 10 mg Oxaliplatin/ml Wasser zu erzielen, sind löslichkeitssteigernde Maßnahmen erforderlich.

In den nachfolgenden Beispielen wird die Eignung ausgewählter, erfindungsgemäßer Kohlenhydrate und Konzentrationen veranschaulicht.

### Versuchsdesign

Die Herstellung der erfindungsgemäßen Lösungskonzentrate und -lösungen erfolgt in allgemein bekannter Weise, bevorzugt unter Berücksichtigung einer möglicht kurzen Prozessdauer (Standzeit) und kontinuierlicher Einhaltung einer möglichst kühlen wirkstoffschonenden Lösungstemperatur. Dabei werden ca. 80% der erforderlichen Menge des Lösungsmittels (Wasser für Injektionszwecke) im Ansatzbehälter vorgelegt, und der lösungsvermittelnde Hilfsstoff bei 20 - 25°C aufgelöst. Anschließend werden die Ansatzlösungen auf Temperaturen von 10 - 15°C heruntergekühlt. Gegebenenfalls können weitere Zusatz- und/oder Hilfsstoffe, wie Antioxidantien, weitere Lösungsvermittler, weitere Lösungsmittel, Konservierungsmittel etc., zugesetzt werden. Auch eine pH-Einstellung durch Säuren, Basen oder Puffer kann in diesem Schritt erfolgen.

Nach der so erfolgten Bereitstellung des erfindungsgemäßen Lösungsmittels wird die entsprechende Menge Oxaliplatin zugegeben und in der Ansatzlösung aufgelöst. Die Ansätze werden mit Wasser für Injektionszwecke (bevorzugt gekühlt auf Temperaturen zwischen 10 und 15°C) auf das erwünschte Endvolumen aufgefüllt, und die so erhaltenen Lösungen ebenfalls bei erniedrigten Temperaturen (bevorzugt bei T = 10 - 15°C) durch einen Sterilfilter in eine sterile Vorlage filtriert. Die aseptische Abfüllung erfolgt in geeignete sterile, dicht verschließbare Behältnisse, z.B. Durchstechflachen mit Injektions- bzw. Infusionsstopfen.

Die Lagerung der gebrauchsfertigen Lösungskonzentrate erfolgt bevorzugt bei 2 - 8°C.

In den Untersuchungen zu vorliegender Erfindung konnte gezeigt werden, dass durch Einsatz von bestimmten wässrigen Kohlenhydratlösungen, die Löslichkeit des Wirkstoffs Oxaliplatin so weit verbessert wird, dass die Auflösung des Wirkstoffs und damit die eigentliche Herstellung des Therapeutikums bei niedrigeren Temperaturen, also wirkstoffschonender als aus dem Stand der Technik bekannt, vorgenommen werden kann. Zudem wird die Löslichkeit des Wirkstoffs so weit verbessert, dass mit den erfindungsgemäßen Lösungen Oxaliplatinlösungskonzentrate zur Verfügung gestellt werden, die einerseits bei weitem einfacher und ungefährlicher zu verwenden sind als die aus dem Stand der Technik bekannten Lyophilisate und andererseits eine langfristige Lagerung in kostengünstigen kleinen Gebinden erlauben. Die erfindungsgemäßen Oxaliplatinlösungskonzentrate zeigen eine völlig unerwartet hohe Langzeitstabilität, ohne die Zugabe pharmazeutisch bedenklicher Zusätze.

### Beispiel 1 - Löslichkeitsverbesserung durch Kohlenhydrat-Zusatz

In Versuchen zur Löslichkeitserhöhung von Oxaliplatin in wässriger Lösung bei Zugabe von größeren Glucoseanteilen bzw. von anderen Kohlenhydraten (beispielhaft: Maltose, Fruktose, Galaktose, Dextran 10, Dextran 70 und Dextran 40) in höherer Konzentrationen (von 20 bis 50%) zeigte sich völlig überraschend, dass in solchen Fällen konzentriertere Oxaliplatinlösungen mit einem Gehalt von weit mehr als 10 mg/ml erhalten werden können. Solche Konzentrate sind auch bei 2 - 8° C über große Zeiträume ohne Kristallbildung beständig, wobei in diesen ersten Experimenten Glucoselösungen Anwendung fanden, die einen Gehalt von 20%, 30% bzw. 40% Glucose (entsprechend 22%, 33% oder 44% Glucose-Monohydrat) bei einem unbeeinflussten pH-Wert aufweisen (siehe Tabelle 4).

In Tabelle 4 sind die Ergebnisse der in diesem Zusammenhang durchgeführten Experimente zusammengefasst. Dabei erfolgte die Herstellung der gesättigten Lösungsüberstände und ihre Zubereitung zur Analyse wie im europäischen Patent EP 1 207 875 beschrieben. In den Löseversuchen erfolgte keine pH-Einstellung (pH-Wert unbeeinflusst). Die Gehaltsbestimmung erfolgte mittels HPLC.

**Tabelle 4: Abhängigkeit der Löslichkeit von Oxaliplatin in wässrigen Lösungen vom zugesetzten Kohlenhydrat und dessen Konzentration in der Lösung.**

| Kohlenhydratanteil | Kohlenhydrat | Oxaliplatin-Konzentration der gesättigten Lösung | pH-Wert der gesättigten Lösung |
|---|---|---|---|
| 0% | Glucose | 7,66 mg/ml | pH 6,1 |
| 5% | Glucose | 8,14 mg/ml | pH 5,9 |
| 10% | Glucose | 10,74 mg/ml | pH 5,8 |
| 20% | Glucose | 11,85 mg/ml | pH 5,7 |
| 30% | Glucose | 12,66 mg/ml | pH 5,6 |
| 40% | Glucose | 13,54 mg/ml | pH 5,6 |
| 50% | Glucose | 14,23 mg/ml | pH 5,4 |
| 50% | Maltose | 17,83 mg/ml | pH 3,7 |
| 50% | Fruktose | 12,56 mg/ml | pH 4,7 |
| 25% | Galaktose | 11,53 mg/ml | pH 6,3 |
| 25% | Dextran 10 | 10,90 mg/ml | pH 4,8 |
| 25% | Dextran 70 | 10,90 mg/ml | pH 4,1 |
| 25% | Dextran 40 | 10,85 mg/ml | pH 4,2 |

In diesen Versuchen erwiesen sich Glucose, Maltose, Fruktose, Galaktose und Dextrane (10 bis 70) als besonders geeignete Löslichkeitsverbesserer zur Erzielung von Oxaliplatinkonzentrationen oberhalb 7 mg/ml Oxaliplatin.

In den durchgeführten Versuchen zur Bestimmung der Eignung und der maximalen Löslichkeit von Oxaliplatin in hydroxylderivathaltigen wässrigen Lösungen erwiesen sich Glucose, Maltose, Fruktose, Galaktose, Dextran 10, Dextran 40 und Dextran 70 als besonders geeignete Hilfsstoffe zum Erhalt von Konzentraten mit einem Gehalt von mehr als 7 mg Oxaliplatin / ml Lösungsmittel.

Obwohl die vorgenannten Oxaliplatinlösungskonzentrate bereits eine ausreichende Wirkstoff- und Lösungsstabilität zeigen, kann der pH-Wert der erfindungsgemäßen Kohlenhydratlösungen, da er bei den direkt erhaltenen Lösungen noch oberhalb von ca. 5,0 liegt, noch durch Zusatz von Säuren und/oder Puffern auf das Stabilitätsoptimum eingestellt werden.

Zur Bestimmung der Wirkstoffstabilität der erfindungsgemäßen Konzentrate wurden die Zersetzungsprodukte zeitaufgelöst bestimmt. Die Ergebnisse dieser Messungen sind im folgenden zusammengefasst

### Beispiel 2 - Stabilität der Oxaliplatin-Lösungskonzentrate

Zur Überprüfung der Stabilität der Lösungskonzentrate wurden entsprechende Oxaliplatinlösungen (c = 10 mg/ml) mit Glucoselösungen unterschiedlicher Konzentration als Lösungsmittel angesetzt, wobei die Auflösung des Wirkstoffs unter Erwärmung der Lösung auf 40 - 45°C über 30 min erfolgte. Die so erhaltenen Lösungen wurden lichtgeschützt bei 25 bzw. 60°C aufbewahrt, und die Anteile der platinhaltigen Zersetzungsprodukte **II** und **III** des Oxaliplatins mittels HPLC bestimmt. Aus diesen Daten ließ sich der Gehalt des Zersetzungsproduktes Oxalsäure errechnen. Die Anteile der Zersetzungsprodukte der erfindungsgemäßen Lösungen sind zeitaufgelöst für die beiden Lagertemperaturen in der Tabelle 5 wiedergegeben.

**Tabelle 5: Stabilität der Oxaliplatinlösungskonzentrate unter beschleunigten Bedingungen; pH-Wert nicht eingestellt (pH = 5,6 ± 0,4). Zersetzungsprodukte bestimmt mittels HPLC.**

| Prüflösung konz. Oxaliplatin Lösungsmittel | Prüftakt | Oxalsäure | Diaquo-DACH-Platin (II) **(II)** | Diaquo-DACH-Platin (II)-Dimer **(III)** |
|---|---|---|---|---|
| c = 10 mg/ml | Start^{a} | 0,242% | 0,117% | not det. |
| 10% Glucose | 1d-25°C | 0,675% | 0,283% | < 0,01% |
| | 1d-60°C | 0,795% | 0,275% | 0,049% |
| c = 10 mg/ml | Start^{a} | 0,200% | 0,110% | not det. |
| 20% Glucose | 1d-25°C | 0,618% | 0,300% | < 0,01% |
| | 1d-60°C | 0,779% | 0,255% | 0,029% |
| c = 10 mg/ml | Start^{a} | 0,234% | 0,117% | not det. |
| 30% Glucose | Id-25°C | 0,646% | 0,300% | < 0,01% |
| | 1d-60°C | 0,799% | 0,273% | < 0,01% |
| c = 10 mg/ml | Start^{a} | 0,212% | 0,125% | not det. |
| 40% Glucose | 1d-25°C | 0,432% | 0,294% | < 0,01% |
| | 1d-60°C | 0,806% | 0,246% | < 0,01% |
| c = 10 mg/ml | Start^{a} | 0,193% | 0,119% | not det. |
| 50% Glucose | Id-25°C | 0,572% | 0,292% | not det. |
| | 1d-60°C | 0,648% | 0,234% | not det. |

| | | | | |
|---|---|---|---|---|
| a) Die Herstellung der Lösungen erfolgte unter Erwärmung der Lösung auf 40 - 45°C über 30 min; die Einlagerung erfolgte unverzüglich bei 20 - 25°C bzw. 60°C. | | | | |

Wie Tabelle 5 zu entnehmen ist, ist bereits durch eine Auflösung bei erhöhter Temperatur, wie sie zur Herstellung konzentrierter Oxaliplatinlösungen aus dem Stand der Technik (z.B c = 5 mg/ml ohne Zusatz von lösungsvermittelnden Substanzen) notwendig ist, eine erhebliche Zersetzung des Wirkstoffs zu beobachten. Diese Zersetzung nimmt mit der Zeit und höherer Temperatur zu; sie wird aber, besonders bei sehr hohen Konzentrationen, durch den Zusatz eines Kohlenhydrats (hier: Glucose) reduziert.

**Tabelle 6: Langzeit-Stabilität der Oxaliplatinlösungskonzentrate bei reduzierten Temperaturen; pH-Wert nicht eingestellt (pH = 5,6 ± 0,2).. Zersetzungsprodukte bestimmt mittels HPLC**

| Prüflösung konz. Oxaliplatin Lösungsmittel | Prüftakt | Oxalsäure | Diaquo-DACH-Platin (II) **(II)** | Diaquo-DACH-Platin (II)-Dimer **(III)** |
|---|---|---|---|---|
| | Start^{a} | 0,21% | 0,105% | < 0,01% |
| c = 10 mg/ml | 12mon-(2-8°C) | 0,32% | 0,152% | 0,029% |
| 20% Glucose | Start^{b} | 0,16% | 0,060% | < 0,01% |
| | 12mon-(2-8°C) | 0,22% | 0,083% | <0,01% |
| | Start^{a} | 0,20% | 0,101% | <0,01% |
| c = 10 mg/ml | 12mon-(2-8°C) | 0,33% | 0,145% | <0,01% |
| 30% Glucose | Start^{b} | 0,19% | 0,072% | <0,01% |
| | 12mon-(2-8°C) | 0,26% | 0,098% | <0,01% |
| | Start^{a} | 0,22% | 0,103% | <0,01% |
| c = 10 mg/ml | 12mon-(2-8°C) | 0,31% | 0,146% | <0,01% |
| 40% Glucose | Start^{b} | 0,17% | 0,064% | <0,01% |
| | 12mon-(2-8°C) | 0,29% | 0,091 % | <0,01% |
| | Start^{a} | 0,20% | 0,106% | < 0,01% |
| c = 10 mg /ml | 12mon-(2-8°C) | 0,30% | 0,150% | <0,01% |
| 50% Glucose | Start^{b} | 0,20% | 0,060% | <0,01% |
| | 12mon-(2-8°C) | 0,28% | 0,109% | <0,01% |

| | | | | |
|---|---|---|---|---|
| a) Die Herstellung der Lösungen erfolgte bei 20 - 25°C; die Einlagerung erfolgte unverzüglich bei 2 - 8°C. b) Die Herstellung der Lösungen erfolgte bei 10 - 15°C; die Einlagerung erfolgte unverzüglich bei 2 - 8°C. | | | | |

Zur Überprüfung, ob sich durch eine schonende Herstellung bei erniedrigten Temperaturen, wie sie erst durch die vorliegende Erfindung ermöglicht wird, und eine kühle Lagerung (2 - 8 °C) langzeitstabile Oxaliplatinlösungskonzentrate erhalten lassen, wurden zeitaufgelöste Messungen an entsprechend hergestellten und aufbewahrten Versuchschargen vorgenommen, deren Ergebnisse in der Tabelle 6 zusammengefasst sind.

Mit den in Tabelle 6 zusammengefassten Experimenten konnte somit gezeigt werden, dass es die Verwendung von Kohlenhydratlösungen (z.B. Glucose Monohydrat in Wasser) als Lösungsmittel zur Herstellung von Oxaliplatinlösungskonzentraten erlaubt, langzeitstabile Injektionskonzentrate bereitzustellen. Die Möglichkeit, diese Konzentrate ohne Erwärmung herzustellen, reduziert die Zersetzung des ansonsten in wässrigen Lösungen höchst instabilen Oxaliplatins soweit, dass selbst nach einem Jahr Lagerung nicht einmal 0, 1 % des Zersetzungsprodukts **II** und weniger als 0,01 % des Dimers **III** beobachtet werden.

Zur weiteren Stabilisierung der Lösungskonzentrate bietet es sich an, durch den Zusatz von Säuren oder Puffer den pH-Wert und damit die Konzentration der aggressiven Hydroxid-anionen zu reduzieren. Zur Überprüfung der Vermutung, dass der Zusatz einer Säure, deren Anion Oxaliplatin nicht destabilisiert, zu den erfindungsgemäßen Oxaliplatinlösungskonzentraten mit Kohlenhydratlösungen als Lösungsmittel die Zersetzung des Wirkstoffs weiter vermindert, wurden exemplarisch die Effekte eines Phosphorsäurezusatzes getestet. Unter identischen Bedingungen wie in den in Tabelle 6 zusammengefassten Messungen wurden die Zersetzungsprodukte von Oxaliplatinlösungskonzentraten (C = 10 mg/ml), deren pH-Wert mit 1N Phosphorsäure auf 3,8 ± 0,2 eingestellt wurde, zeitaufgelöst und quantitativ mittels HPLC bestimmt. Die Ergebnisse dieser Experimente sind in Tabelle 7 zusammengefasst.

**Tabelle 7: Langzeit-Stabilität der Oxaliplatinlösungskonzentrate bei reduzierten Temperaturen; pH-Wert voreingestellt mit 1N Phosphorsäure (pH = 3,8 0,2). Zersetzungsprodukte bestimmt mittels HPLC**

| Prüflösung konz. Oxaliplatin Lösungsmittel | Prüftakt | Oxalsäure | Diaquo-DACH-Platin (II) **(II)** | Diaquo-DACH-Platin (II)-Dimer **(III)** |
|---|---|---|---|---|
| | Start^{a} | 0,18% | 0,093% | <0,01% |
| c = 10 mg/ml | 12mon-(2-8°C) | 0,28% | 0,141% | 0,020% |
| 20% Glucose | Start^{b} | 0,14% | 0,052% | < 0,01% |
| | 12mon-(2-8°C) | 0,20% | 0,098% | <0,01% |
| | Start^{a} | 0,19% | 0,100% | <0,01% |
| c = 10 mg/ml | 12mon-(2-8°C) | 0,30% | 0,138% | <0,01% |
| 30% Glucose | Start^{b} | 0,13% | 0,052% | < 0,01% |
| | 12mon-(2-8°C) | 0,20% | 0,098% | <0,01% |
| | Start^{a} | 0,20% | 0,092% | <0,01% |
| c = 10 mg/ml | 12mon-(2-8°C) | 0,28% | 0,126% | <0,01% |
| 40% Glucose | Start^{b} | 0,14% | 0,054% | < 0,01% |
| | 12mon-(2-8°C) | 0,21% | 0,101% | <0,01% |
| | Start^{a} | 0,19% | 0,088% | <0,01% |
| c = 10 mg/ml | 12mon-(2-8°C) | 0,25% | 0,111% | <0,01% |
| 50% Glucose | Start^{b} | 0,16% | 0,058% | < 0,01% |
| | 12mon-(2-8°C) | 0,22% | 0,100% | < 0,01% |

| | | | | |
|---|---|---|---|---|
| a) Die Herstellung der Lösungen erfolgte bei 20 - 25°C; die Einlagerung erfolgte unverzüglich bei 2 - 8°C. b) Die Herstellung der Lösungen erfolgte bei 10 - 15°C; die Einlagerung erfolgte unverzüglich bei 2 - 8°C. | | | | |

Wie den in Tabelle 7 zusammengefassten Ergebnissen zu entnehmen ist, lassen sich die Lösungskonzentrate durch die Reduktion des pH-Werts durch Zusatz einer Säure, deren Anion Oxaliplatin nicht destabilisiert, noch weiter stabilisieren.

Damit lässt sich den unter Beispiel 2 (Tabellen 5-7) zusammengefassten Experimenten unzweifelhaft entnehmen, dass durch die Verwendung von bestimmten Kohlenhydratlösungen als Lösungsmittel in der Herstellung von Oxaliplatinlösungskonzentraten langzeitstabile pharmazeutische Präparate zu erhalten sind. Des weiteren erlaubt die Verwendung der erfindungsgemäßen Lösungsmittel Oxaliplatinlösungen besonders schonend herzustellen. Schließlich wirkt sich auch der Zusatz von Säuren, die den Wirkstoff Oxaliplatin nicht destabilisieren (z.B. etwa Phosphor- oder Schwefelsäure), auch in den erfindungsgemäßen Konzentraten positiv aus.

### Beispiele 3 - 69 - Zusammensetzung exemplarischer erfindungsgemäßer Oxaliplatinlösungskonzentrate

Im folgenden sind Zusammensetzungen der erfindungsgemäßen Oxaliplatinlösungskonzentrate exemplifiziert. (Nur No 6, 27-29 und 67-69 sind Anteil der Erfindung).

**Tabelle 8: pH-eingestellte erfindungsgemäßen Oxaliplatin Lösungskonzentrate**

| No | Oxaliplatin (mg/ml) | pH-Wert | Säure | Kohlenhydrat, Hydroxyderivat | Pufferbase |
|---|---|---|---|---|---|
| 3. | 7,5 | 3,2 | Phosphorsäure | | |
| 4. | 7,5 | 3,2 | Phosphorsäure | 10 mg/ml Glucose Monohydrat | |
| 5. | 8 | 3,2 | Phosphorsäure | 10 mg/ml Glucose Monohydrat | |
| 6. | 8 | 3,2 | Phosphorsäure | 10 mg/ml Fruktose | |
| 7. | 8 | 3,2 | Phosphorsäure | | |
| 8. | 8 | 3,2 | Schwefelsäure | | |
| 9. | 8 | 3,0 | Phosphorsäure | 10 mg/ml Glucose Monohydrat, 100 mg/ml Polyethylenglycol 400 | 5 mg/ml Dinatriumhydrogenphosphat |
| 10. | 8 | 3,3 | Phosphorsäure | 100 mg/ml Polyethylenglycol 600 | 2 mg/ml Dinatriumhydrogenphosphat |
| 11. | 8 | 3,5 | Milchsäure | 100 mg/ml Polyethylenglycol 600 | 2 mg/ml Dinatriumhydrogenphosphat |
| 12. | 8 | 3,5 | Phosphorsäure | 100 mg/ml Laktose Monohydrat | |
| 13. | 10 | 3,0 | Phosphorsäure | 100 mg/ml Glucose Monohydrat | |
| 14. | 10 | | | 150 mg/ml Glucose Monohydrat | |
| 15. | 10 | | | 200 mg/ml Glucose Monohydrat | |
| 16. | 10 | | | 250 mg/ml Glucose Monohydrat | |
| 17. | 10 | | | 300 mg/ml Glucose Monohydrat | |
| 18. | 10 | | | 350 mg/ml Glucose Monohydrat | |
| 19. | 10 | | | 400 mg(ml Glucose Monohydrat | |
| 20. | 10 | | | 450 mg/ml Glucose Monohydrat | |
| 21. | 12 | | | 500 mg/ml Glucose Monohydrat | |
| 22. | 12 | | | 600 mg/ml Glucose Monohydrat | |
| 23. | 12 | | | 700 mg/ml Glucose Monohydrat | |
| 24. | 15 | | | 800 mg/ml Glucose Monohydrat | |
| 25. | 20 | | | 900 mg/ml Glucose Monohydrat | |
| 26. | 10 | 3,0 | Phosphorsäure | 150 mg/ml Glucose Monohydrat | |
| 27. | 10 | 3,0 | Phosphorsäure | 200 mg/ml Maltose Monohydrat | |
| 28. | 10 | 3,2 | Phosphorsäure | 350 mg/ml Maltose Monohydrat | |
| 29. | 12 | 3,4 | Phosphorsäure | 500 mg/ml Maltose Monohydrat | |
| 30. | 10 | 3,0 | Phosphorsäure | 150 mg/ml Glucose Monohydrat | 2,5 mg/ml Dinatriumhydrogenphosphat |
| 31. | 10 | 2,9 | Phosphorsäure | 200 mg/ml Glucose Monohydrat | |
| 32. | 10 | 3,2 | Phosphorsäure | 200 mg/ml Glucose Monohydrat | |
| 33. | 10 | 3,6 | Phosphorsäure | 200 mg/ml Glucose Monohydrat | |
| 34. | 10 | 3,8 | Phosphorsäure | 200 mg/ml Glucose Monohydrat | |
| 35. | 10 | 4,0 | Phosphorsäure | 200 mg/ml Glucose Monohydrat | |
| 36. | 10 | 4,2 | Phosphorsäure | 200 mg/ml Glucose Monohydrat | |
| 37. | 10 | 3,4 | Phosphorsäure | 200 mg/ml Glucose Monohydrat | 0,25 mg/ml Dinatriumhydrogenphosphat |
| 38. | 10 | 3,6 | Phosphorsäure | 300 mg/ml Glucose Monohydrat | 0,25 mg/ml Dinatriumhydrogenphosphat |
| 39. | 10 | 3,8 | Phosphorsäure | 400 mg/ml Glucose Monohydrat | 0,15 mg/ml Dinatriumhydrogenphosphat |
| 40. | 10 | 4,0 | Phosphorsäure | 500 mg/ml Glucose Monohydrat | 0,25 mg/ml Dinatriumhydrogenphosphat |
| 41. | 10 | 3,2 | Schwefelsäure | 200 mg/ml Glucose Monohydrat | |
| 42. | 10 | 3,4 | Schwefelsäure | 200 mg/ml Glucose Monohydrat | 0,25 mg/ml Dinatriumhydrogenphosphat |
| 43. | 10 | 3,6 | Schwefelsäure | 300 mg/ml Glucose Monohydrat | 0,25 mg/ml Dinatriumhydrogenphosphat |
| 44. | 10 | 3,8 | Schwefelsäure | 400 mg/ml Glucose Monohydrat | 0,25 mg/ml Dinatriumhydrogenphosphat |
| 45. | 10 | 4,0 | Schwefelsäure | 500 mg/ml Glucose Monohydrat | 25 mg/ml Dinatriumhydrogenphosphat |
| 46. | 10 | 3,4 | Phosphorsäure | 500 mg/ml Glucose Monohydrat | |
| 47. | 10 | 3,8 | Schwefelsäure | 800 mg/ml Glucose Monohydrat | |
| 48. | 10 | 3,9 | Ethansulfonsäur e | 900 mg/ml Glucose Monohydrat | |
| 49. | 10 | 3,5 | Milchsäure | 200 mg/ml Glucose Monohydrat | |
| 50. | 10 | 3,5 | Milchsäure | 200 mg/ml Glucose Monohydrat | 1 mg/ml Natriumlactat |
| 51. | 10 | 3,5 | Milchsäure | 250 mg/ml Glucose Monohydrat | 20 mg/ml Natriumlactat |
| 52. | 10 | 3,5 | Milchsäure | 300 mg/ml Glucose Monohydrat | 2 mg/ml Natriumlactat |
| 53. | 10 | 4,0 | Milchsäure | 400 mg/ml Glucose Monohydrat | 2 mg/ml Natriumlactat |
| 54. | 10 | 3,5 | Phosphorsäure | 200 mg/ml Glucose Monohydrat | |
| 55. | 10 | 3,5 | Phosphorsäure | 500 mg/ml Glucose Monohydrat | |
| 56. | 10 | 3,5 | Phosphorsäure | 800 mg/ml Glucose Monohydrat | |
| 57. | 10 | 3,5 | Phosphorsäure | 900 mg/ml Glucose Monohydrat | |
| 58. | 10 | 6,0 | Phosphorsäure | 100 mg/ml Glucose Monohydrat | |
| 59. | 10 | 6,0 | Phosphorsäure | 200 mg/ml Glucose Monohydrat | |
| 60. | 10 | 6,0 | Phosphorsäure | 500 mg/ml Glucose Monohydrat | |
| 61. | 10 | 6,0 | Phosphorsäure | 800 mg/ml Glucose Monohydrat | |
| 62. | 15 | 2,9 | Phosphorsäure | 900 mg/ml Glucose Monohydrat | |
| 63. | 15 | 6,0 | Phosphorsäure | 900 mg/ml Glucose Monohydrat | |
| 64. | 15 | 6,0 | Phosphorsäure | 900 mg/ml Glucose Monohydrat | |
| 65. | 20 | 3,3 | Phosphorsäure | 900 mg/ml Glucose Monohydrat | |
| 66. | 20 | 3,4 | Phosphorsäure | 700 mg/ml Glucose Monohydrat | |
| 67. | 20 | 3,4 | Phosphorsäure | 700 mg/ml Fruktose Monohydrat | |
| 68. | 15 | 3,4 | Phosphorsäure | 500 mg/ml Fruktose Monohydrat | |
| 69. | 25 | 3,0 | Phosphorsäure | 900 mg/ml Glucose Monohydrat | |

## Patentansprüche

1. Pharmazeutische Zusammensetzung, die Wasser, Oxaliplatin und ein physiologisch verträgliches Kohlenhydrat umfasst, wobei das physiologisch verträgliche Kohlenhydrat ausgewählt ist aus der Gruppe Maltose, Fruktose, Galaktose, Dextran oder Mischungen davon.

2. Pharmazeutische Zusammensetzung nach Anspruch 1, die gegebenenfalls zusätzlich weitere tonisierende, den pH-Wert einstellende, puffernde oder konservierende Hilfsstoffe umfasst.

3. Pharmazeutische Zusammensetzung nach einem der Ansprüche 1 bis 2, wobei die Oxaliplatin-Konzentration zwischen 5 mg/ml und 25 mg/ml liegt.

4. Pharmazeutische Zusammensetzung nach einem der Ansprüche 1 bis 2, wobei die Oxaliplatin-Konzentration zwischen 8 mg/ml und 20 mg/ml liegt.

5. Pharmazeutische Zusammensetzung nach einem der Ansprüche 1 bis 2, wobei die Oxaliplatin-Konzentration höher ist als die maximal lösliche Konzentration in reinem Wasser.

6. Pharmazeutische Zusammensetzung nach einem der Ansprüche 1 bis 5, wobei die Kohlenhydratkonzentration mindestens 50 mg/ml beträgt.

7. Pharmazeutische Zusammensetzung nach einem der Ansprüche 1 bis 5, wobei die Kohlenhydrat-Konzentration oberhalb von 5% (w/v) liegt.

8. Verfahren zur Herstellung der pharmazeutischen Zusammensetzungen nach einem der Ansprüche 1 bis 7, wobei Oxaliplatin bei Temperaturen von 2 - 15°C in einem kohlenhydrathaltigen Lösungsmittel gelöst wird.

9. Pharmazeutische Zusammensetzung, die Wasser, Oxaliplatin und mehr als 5 Gew.-% Glucose umfasst.

10. Pharmazeutische Zusammensetzung nach Anspruch 9, wobei die Oxaliplatin-Konzentration mindestens 10 mg/ml beträgt.

11. Pharmazeutische Zusammensetzung nach einem der Ansprüche 9 oder 10, die im Weiteren eine anorganische oder organische Säure umfasst, wobei das Anion der Säure die Stabilität der Lösung nicht beeinträchtigt.

12. Pharmazeutische Zusammensetzung nach Anspruch 11, wobei die Säure ausgewählt ist aus der Gruppe Phosphorsäure, Schwefelsäure, Methansulfonsäure, Ethansulfonsäure und para-Toluolsulfonsäure oder Mischungen davon.

13. Pharmazeutische Zusammensetzung nach einem der Ansprüche 1 bis 12, die nicht unmittelbar vor der Verabreichung an einen Patienten aus einer festen Oxaliplatin-Zubereitung rekonstituiert wurde.

14. Verwendung der pharmazeutischen Zusammensetzung nach einem der Ansprüche 1 bis 13 zur Herstellung eines Arzneimittels zur Behandlung einer Tumorerkrankung.

15. Verwendung eines physiologisch verträglichen Kohlenhydrats zur Herstellung eines stabilen, pharmazeutisch akzeptablen Lösungskonzentrats von Oxaliplatin in Wasser, wobei das physiologisch verträgliche Kohlenhydrat ausgewählt ist aus der Gruppe Maltose, Fruktose, Galaktose, Dextran oder Mischungen davon.

16. Verwendung eines Kohlenhydrats zur Verbesserung der Löslichkeit von Oxaliplatin in wässrigen Lösungen, wobei das physiologisch verträgliche Kohlenhydrat ausgewählt ist aus der Gruppe Maltose, Fruktose, Galaktose, Dextran oder Mischungen davon.

## Claims

1. Pharmaceutical composition comprising water, oxaliplatin, and a physiologically acceptable carbohydrate, wherein the physiologically acceptable carbohydrate is selected from the group consisting of maltose, fructose, galactose and dextran, or mixtures thereof.

2. A pharmaceutical composition according to claim 1, further comprising additional tonic, pH-adjusting, buffering, or preserving adjuvants.

3. A pharmaceutical composition according to claim 1 or claim 2, wherein the oxaliplatin concentration ranges from 5 mg/ml to 25 mg/ml.

4. A pharmaceutical composition according to claim 1 or claim 2, wherein the oxaliplatin concentration ranges from 8 mg/ml to 20 mg/ml.

5. A pharmaceutical composition according to claim 1 or claim 2, wherein the oxaliplatin concentration is higher than the maximum soluble concentration in pure water.

6. A pharmaceutical composition according to any one of claims 1-5, wherein the carbohydrate concentration is at least 50 mg/ml.

7. A pharmaceutical composition according to any one of claims 1-5, wherein the carbohydrate-concentration is greater than 5 % (w/v).

8. A method for preparing a pharmaceutical composition according to any one of claims 1-7, wherein the oxaliplatin is dissolved at a temperature between 2-15 °C in a carbohydrate containing solvent.

9. A pharmaceutical composition comprising water, oxaliplatin, and more than 5 % glucose (w/w).

10. A pharmaceutical composition according to claim 9, wherein the oxaliplatin concentration is at least 10 mg/ml.

11. A pharmaceutical composition according to claim 9 or claim 10, further comprising an inorganic or organic acid, wherein the anion of the acid does not impair the stability of the solution.

12. A pharmaceutical composition according to claim 11, wherein the acid is selected from the group consisting of phosphoric acid, sulfuric acid, methanesulfonic acid, ethanesulfonic acid and para-toluenesulfonic acid, or mixtures thereof.

13. A pharmaceutical composition according to any one of claims 1-12, wherein the composition is not reconstituted from a solid oxaliplatin preparation immediately before being administered to a patient.

14. Use of the pharmaceutical composition according to any one of claims 1-13 in the manufacture of a pharmaceutical preparation for the treatment of tumor-related illnesses.

15. Use of a physiologically acceptable carbohydrate for the preparation of a stable, pharmaceutically acceptable concentrate of oxaliplatin in water, wherein the physiologically acceptable carbohydrate is selected from the group consisting of maltose, fructose, galactose and dextran, or mixtures thereof.

16. Use of a physiologically acceptable carbohydrate for increasing the solubility of oxaliplatin in aqueous solutions, wherein the physiologically acceptable carbohydrate is selected from the group consisting of maltose, fructose, galactose and dextran, or mixtures thereof.

## Revendications

1. Composition pharmaceutique contenant de l'eau, de l'oxaliplatine et un hydrate de carbone physiologiquement compatible, ledit hydrate de carbone physiologiquement compatible étant choisi dans le groupe formé par le maltose, le fructose, le galactose, le dextrane ou des mélanges de ceux-ci.

2. Composition pharmaceutique selon la revendication 1, contenant éventuellement à titre complémentaire d'autres adjuvants tonifiants, ajustant le pH, à effet tampon ou conservateurs.

3. Composition pharmaceutique selon la revendication 1 ou la revendication 2, dans laquelle la concentration d'oxaliplatine est comprise entre 5 mg/ml et 25 mg/ml.

4. Composition pharmaceutique selon la revendication 1 ou la revendication 2, dans laquelle la concentration d'oxaliplatine est comprise entre 8 mg/ml et 20 mg/ml.

5. Composition pharmaceutique selon la revendication 1 ou la revendication 2, dans laquelle la concentration d'oxaliplatine est supérieure à la concentration maximale soluble dans l'eau pure.

6. Composition pharmaceutique selon l'une quelconque des revendications 1 à 5, dans laquelle la concentration d'hydrate de carbone est supérieure ou égale à 50 mg/ml.

7. Composition pharmaceutique selon l'une quelconque des revendications 1 à 5, dans laquelle la concentration d'hydrate de carbone est supérieure à 5 % (p/v).

8. Procédé de fabrication des compositions pharmaceutiques selon l'une quelconque des revendications 1 à 7, dans lequel l'oxaliplatine est dissoute à une température comprise entre 2 et 15 °C dans un solvant contenant un hydrate de carbone.

9. Composition pharmaceutique contenant de l'eau, de l'oxaliplatine et plus de 5 % en poids de glucose.

10. Composition pharmaceutique selon la revendication 9, dans laquelle la concentration d'oxaliplatine est supérieure ou égale à 10 mg/ml.

11. Composition pharmaceutique selon la revendication 9 ou la revendication 10, contenant en outre un acide inorganique ou organique, l'anion de l'acide n'affectant pas la stabilité de la solution.

12. Composition pharmaceutique selon la revendication 11, dans laquelle l'acide est choisi dans le groupe formé par l'acide phosphorique, l'acide sulfurique, l'acide méthane sulfonique, l'acide éthane sulfonique et l'acide paratoluènesulfonique ou des mélanges de ceux-ci.

13. Composition pharmaceutique selon l'une quelconque des revendications 1 à 12, qui n'est pas reconstituée immédiatement avant l'administration à un patient à partir d'une préparation solide d'oxaliplatine.

14. Utilisation de la composition pharmaceutique selon l'une quelconque des revendications 1 à 13 pour préparer un médicament destiné à traiter les tumeurs.

15. Utilisation d'un hydrate de carbone physiologiquement compatible pour préparer une solution concentrée stable et pharmacologiquement acceptable d'oxaliplatine dans l'eau, ledit hydrate de carbone physiologiquement compatible étant choisi dans le groupe formé par le maltose, le fructose, le galactose, le dextrane ou des mélanges de ceux-ci.

16. Utilisation d'un hydrate de carbone pour améliorer la solubilité de l'oxaliplatine dans les solutions aqueuses, ledit hydrate de carbone physiologiquement compatible étant choisi dans le groupe formé par le maltose, le fructose, le galactose, le dextrane ou des mélanges de ceux-ci.
